# EUROPEAN PATENT SPECIFICATION

(11) **EP 4 301 739 B1**
(45) Date of publication and mention of the grant of the patent: **23.04.2025**
(21) Application number: 22714240.3
(22) Date of filing: 02.03.2022
(51) Int. Cl.: C07D 295/185, C07D 211/38, C07D 223/12, C07D 209/44, C07D 209/08, C07D 471/04, C07D 217/06, C07D 215/08, A61K 31/40, A61K 31/4015, A61K 31/4035, A61K 31/404, A61K 31/445, A61K 31/4465, A61K 31/47, A61K 31/472, A61P 25/00

(54) **FPR2 (FORMYL PEPTIDE RECEPTOR 2) RECEPTOR AGONISTS AND THEIR USE IN THE TREATMENT OF THE AUTISM SPECTRUM DISORDER**
FPR2 (FORMYL PEPTIDE RECEPTOR 2) REZEPTOR AGONISTEN UND IHRE VERWENDING FÜR DIE BEHANDLUNG VON AUTISM SPEKTRUM STÖRUNG
AGONISTES DE LA FPR2 (FORMYL PEPETIDE RECEPTOR 2) ET LEUR UTILISATION POUR LA TRAITEMENT DU DÉSORDRE DE LA SPECTRUM DE L'AUTISME

(30) Priority: 03.03.2021 IT 202100004964
(43) Date of publication of application: 10.01.2024
(73) Proprietor: Università degli Studi di Bari "Aldo Moro", 70121 Bari (BA) (IT)
(72) Inventor: LEOPOLDO, Marcello, 70125 Bari (IT); LACIVITA, Enza, 70125 Bari (IT); MASTROMARINO, Margherita, 70125 Bari (IT); PERRONE CAPANO, Carla, 80131 Napoli (IT); VOLPICELLI, Floriana, 80131 Napoli (IT); CRISPINO, Marianna, 80126 Napoli (IT); CRISTIANO, Claudia, 80131 Napoli (IT); CALIGNANO, Antonio, 80131 Napoli (IT)
(74) Representative: Trupiano, Federica
(86) International application number: PCT/IB2022/051832
(87) International publication number: WO 2022/185227

(56) References cited:
- WO-A1-2012/102832
- WO-A2-2005/047899
- US-A1- 2014 256 684
- YAN , C-L ET.AL.: "Decreased Plasma Levels of Lipoxin A4 in Children with Autistic Spectrum Disorders.", NEUROREPORT, vol. 26, no. 6, 15 January 2015 (2015-01-15), pages 341 - 345, XP055834382, DOI: 10.1097/WNR.0000000000000350
- O. CORMINBOEUF ET. AL.: "FPR2/ALXR Agonists and the Resolution of Inflammation", JOURNAL OF MEDICINAL CHEMISTRY, vol. 58, no. 2, 14 November 2014 (2014-11-14), pages 537 - 559, XP002804155, DOI: 10.1021/jm501051x

## Description

### Summary of the invention

The present invention relates to novel Formyl Peptide Receptor subtype 2 (FPR2) agonist compounds and their use in the recovery of the inflammatory phenomena in which said receptor is involved, particularly in the context of the treatment of the autism spectrum disorder.

### Background art

The Autism Spectrum Disorder (ASD) is a neurodevelopmental disorder characterized by three characteristic symptoms: affective isolation or inability to relate adequately to people, restricted repertoire of interests or very rigid patterns of action, problems with both verbal and non-verbal communication and imagination. The underlying causes of ASD are not known but it is believed that they may have a component of genetic origin and/or environmental origin in varying proportions ranging from 0 to 100%.

Currently, there are very few drugs approved for the treatment of this disorder, in particular they are anti-psychotic molecules such as aripiprazole and risperidone, which act on some symptoms of the disease, by reducing aggressiveness, irritability, and stereotypies, but that do not have an effect on what are other fundamental aspects that characterize the ASD, such as the affective isolation, the inability to relate adequately with people, a restricted repertoire of interests or very rigid patterns of action, verbal and non-verbal communication problems, as well as poor imaginative ability.

*Post mortem* studies performed on the brains of patients affected by ASD allowed to bring to light the presence of neuroinflammatory phenomena that could be the cause or contributory cause of the neuronal defects found at the microscopic level and the consequent symptoms that characterize said disorder.

In fact, it has been found that some neurodegenerative diseases, such as Alzheimer's disease and Parkinson's disease, are associated with neuroinflammatory phenomena that may constitute, at least in part, the cause of the manifestations of these diseases.

Physiologically, the recovery of the inflammation is mediated by endogenous molecules called *"Specialized Pro-resolving Mediators"* (SPM) which, as the name denotes, have the role of ending the inflammatory phase by acting on specific receptors that aim to restore the tissue homeostasis after an inflammatory event. Among the SPMs, lipoxin A4 (LXA4) is one of the major mediators of the inflammation recovery and exerts its function through the interaction with the *Formyl Peptide Receptor* subtype 2 (FPR2).

WO 2005/047899 discloses compounds that selectively activate the FPRL1 receptor in order to alleviate inflammatory responses and to treat inflammation and pain.

WO 2012/102832 refers to compounds, compositions and methods for treating Autism Spectrum Disorders (ASD) using glycyl-2-methylprolyl-glutamic acid (G-2-MePE) and analogs thereof.

US 2014/256684 discloses some N-phenyl-N'-phenylethyl ureas compounds, substituted on the phenylethyl by a carboxy group, as FPR2 modulators and, in particular, for the treatment of ocular inflammation.

Yan, C-L et al. (NeuroReport, vol. 26(6), 2015) disclose a link between decreased lipoxin A4 levels and autism in children suggesting an increased susceptibility to recurring inflammation. The authors hypothesized that following lipoxin A4 levels over time may enable prediction of which children will develop autism and allow for earlier treatment interventions.

Corminboeuf, O et al., (J. Med Chelm, vol. 58 (2), 2014) is a perspective study which gives a general review of some known compounds with FPR/ALXR modulator activity, and described as helpful in the resolution of inflammation.

It is currently felt the need to provide novel compounds that are able to act on the ASD and that comprise the ability to obtain improvements even at the level of those aspects more related to affectivity and imagination, currently not treatable with antipsychotic drugs used today in the therapy of the autism spectrum disorder.

### Objects of the invention

It is an object of the present invention to provide novel compounds capable of binding to the FPR2 receptor, and in particular of stimulating its activation.

It is a further object of the present invention the use of said compound in therapy, particularly for the treatment of the autism spectrum disorder.

These and other objects are achieved by the subject matter of the present invention which provides a novel FPR2 receptor agonist compound.

### Description of the figures

Figure 1: results obtained in the *three-chambered social test* (Figure 1A) and in the *reciprocal social interactions test* (Figure 1B) following the systemic administration of (S)-16 for 8 days at a dose of 10 mg/kg (gray histogram) compared with control subjects who were administered the vehicle only without active molecule (white histogram).
Figure 2: levels of FPR2 (left graph), interleukin 1 beta (IL-1β) (middle graph), and tumor necrosis factor alpha (TNF-α) (right graph) following the systemic administration of (S)-16 for 8 days at a dose of 10 mg/kg compared with C57 or BTBR control mice who were administered the vehicle only without active molecule.
Figure 3: neurite length of hippocampal neurons from C57 wild type (wt) mice or BTBR mice in the absence or following systemic administration of (S)-16 for 4 or 72 hours.
Figure 4: effect of the compounds (S)-4, (S)-5, (S)-7, (R)-8, (S)-8, (S)-9, (S)-12, (S)-15 and (S)-16 on the cell viability (LDH assay) in cultures of mouse microglial N9 cells in the presence and absence of stimulation with LPS.
Figure 5: effect of the compounds (S)-4, (S)-7, (S)-8, (S)-9 and (S)-16 on the IL-1β release in cultures of mouse microglial N9 cells in the presence and absence of stimulation with LPS.
Figure 6: effect of the compounds (S)-4, (S)-7, (S)-8, (S)-9 and (S)-16 on the TNF-α release in cultures of mouse microglial N9 cells in the presence and absence of stimulation with LPS.

### Description of the invention

A study performed on a cohort of children affected by ASD highlighted the presence of low blood levels of lipoxin A4 (LXA4); this observation is the basis of the hypothesis formulated by the inventors regarding the possibility of intervening on the neuroinflammatory phenomena, potentially involved in autism spectrum disorder, by stimulating the activation of the FPR2 receptor. Said stimulation will have to occur through the use of a synthetic molecule capable of interacting with the receptor of interest, which is resistant to the systemic metabolism and has the ability to reach the central nervous system, the main target of its action.

Thus, object of the present invention, according to one aspect thereof, is the compound of general formula (I) 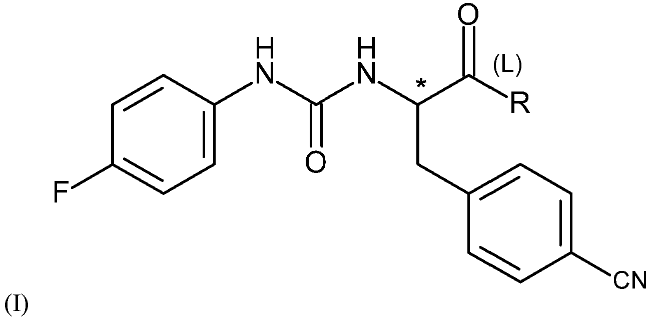 wherein R is selected from one of the following groups listed in Table 1, each directly bonded to the C=O group of the compound of formula (I) via the bond (L) by the nitrogen atom denoted by the letter (a) in Table 1 below, and the chiral carbon, denoted by the asterisk, may be in the S and/or R configuration.

**Table 1: meaning of the R group, wherein (a) denotes the nitrogen atom engaged in bonding to the compound of formula (I) and (L) denotes the covalent bond binding the compound of formula (I) to each group R, and abbreviation which identified the corresponding compound.**

| Abbreviation | Meaning of R |
|---|---|
| 1 | 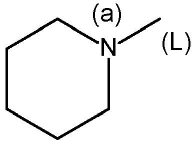 |
| 2 | 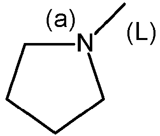 |
| 3 | 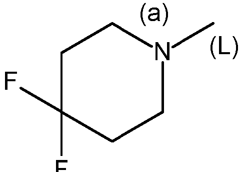 |
| 4 | 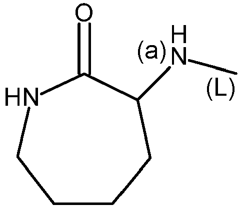 |
| 5 | 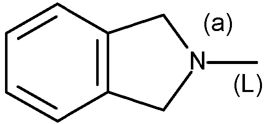 |
| 6 | 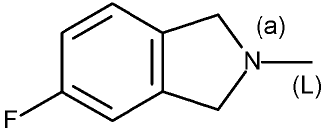 |
| 7 | 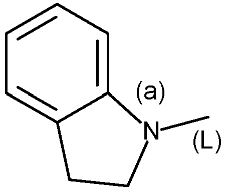 |
| 8 | 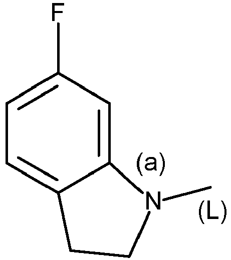 |
| 9 | 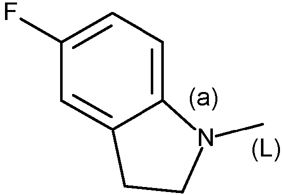 |
| 10 | 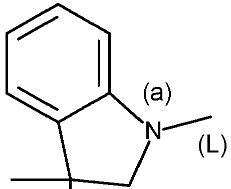 |
| 11 | 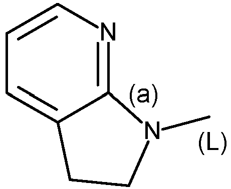 |
| 12 | 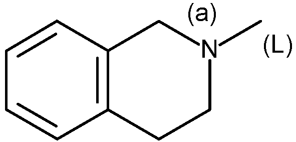 |
| 13 | 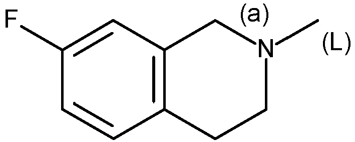 |
| 14 | 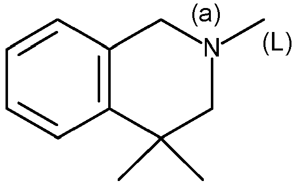 |
| 15 | 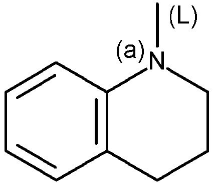 |
| 16 | 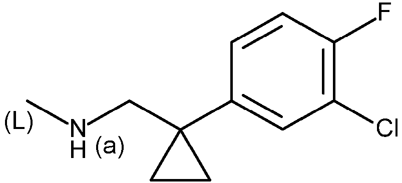 |

Preferred are the compounds of formula (I) and the R-group meanings listed in Table 1 in their chiral form S.

Particularly preferred are the compounds 4, 7, 8 and 9, preferably in the chiral form S with respect to carbon denoted by the asterisk in the general formula (I).

Another particularly preferred compound is the compound 16, particularly preferred is its chiral form S with respect to carbon denoted by the asterisk in the general formula (I).

In the present description, when we wish to refer to a specific chiral or racemic form of the carbon denoted by the asterisk in the general formula (I) of one of the compounds listed in Table 1, the letters S, R, or both, in brackets, will precede the abbreviation denoting the compound. For illustrative purpose: (S)-16 denotes the S form of the compound 16; (S,R)-12 denotes the racemic form of the compound 12; (R)-8 denotes the R form of the compound 8.

The compounds of the invention are capable of exerting an activating action on the FPR2 receptor, accompanied by an amelioration of the pathological behaviors determined by the ASD, as will be shown in the Experimental section.

The compounds of the present invention may be synthesized according to the conventional methods known to the skilled in the art.

For example, a possible synthetic route of the compound 16 is depicted in the following scheme (I):

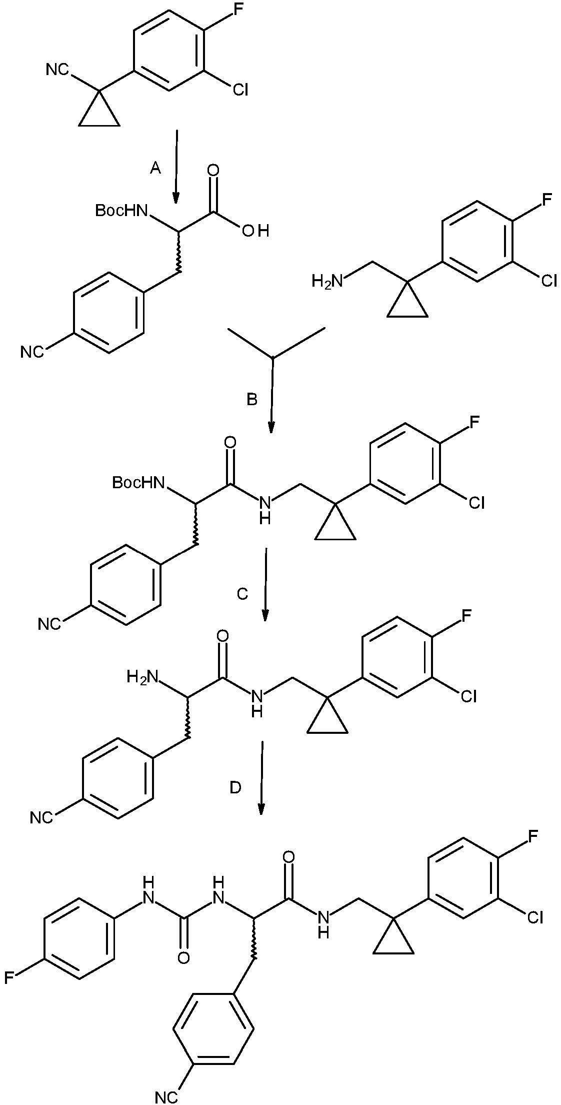

Scheme (I), reagents, and conditions: (A) 10 M borane-methyl sulfide complex, reflux, 5 hours; 3N HCl, reflux, 1 hour; (B) N,N'-carbonyldiimidazole, room temperature, overnight; (C) trifluoroacetic acid, room temperature, 5 hours; (D) 4-phenylisocyanate, room temperature, overnight.

The bond depicted with 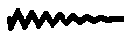 may be in the S and/or R configuration.

Another object of the present invention is the use of the novel compounds of formula (I), and particularly of the preferred compounds, as activators of FPR2.

Further object of the present invention is the use of the novel compounds of formula (I), and particularly of the preferred compound, in the treatment of autism spectrum disorder.

For their use in therapy, the compounds of the invention are formulated in pharmaceutical compositions together with at least one pharmaceutically acceptable carrier according to methods known to the skilled in the art; said compositions constitute another aspect of the present invention.

The skilled in the art, once the chemical-physical characteristics and bioavailability of the compound to be administered are known, is able to select the most suitable formulation, by selecting according to the intended route of administration. The compositions of the invention may provide for intravenous, intraperitoneal, subcutaneous, intramuscular and/or oral administration.

The compositions of the invention may also comprise, in addition to suitable excipients, additional active ingredients. Non-limiting examples of possible additional active ingredients are represented by the molecules that have a positive effect on the LXA4 biosynthesis, particularly acetylsalicylic acid.

The present invention, according to another aspect thereof, relates to a method of treating diseases involving an insufficient activation of the FPR2 receptor, which comprises administering an effective amount of the compound of formula (I) to the patient in the need thereof. In particular, said method is preferably aimed at the treatment of the autism spectrum disorder.

In the Experimental section below, the laboratory evidence demonstrating the agonist activity at the FPR2 receptor of the novel compounds of the invention and in particular in a murine model of autism, through behavioral and biochemical studies, will be described in a non-limiting way.

### Experimental section

### Synthesis of the compound (S)-4

### Intermediate a) (S)-tert-Butyl (3-(4-cyanophenyl)-1-oxo-1-((2-oxoazepan-3-yl)amino)propan-2-yl)carbamate.

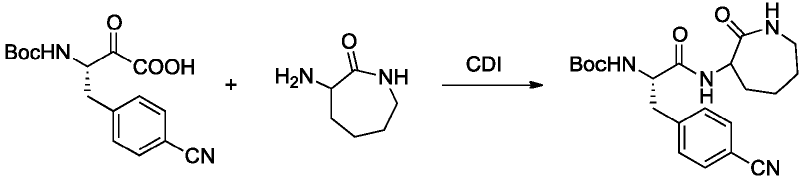

To a solution of (S)-Boc-4-CN-phenylalanine (0.25 g, 0.86 mmol) in anhydrous THF (10 mL), *N'*,*N*-carbonyldiimidazole (0.154 g, 0.95 mmol) is added and the reaction mixture is stirred at room temperature overnight. Then, a solution of DL-α-amino-ε-caprolactam (0.11 g, 0.86 mmol) is added and the mixture is stirred for another 24 hours at room temperature. After completion, the solvent is concentrated under reduced pressure and the residue is partitioned between AcOEt and H₂O and extracted with AcOEt (3 × 20 mL). The collected organic phases are dried over anhydrous Na₂SO₄, filtered and concentrated under reduced pressure. The reaction crude is purified by silica gel chromatography, using a CHCl₃/MeOH mixture (95:5, v/v) as eluent to achieve the desired compound as a white solid (56% yield). ¹H-NMR (CDCl₃): δ 1.36 (s, 9H), 1.68-2.00 (m, 6H), 3.04-3.23 (m, 4H), 4.41-4.45 (m, 2H), 5.10-5.29 (dd, NH, 1H), 6.17 (s, NH, 1H), 6.33 (d, 1H, NH, *J*=1.8 Hz), 7.55 (d, 2H, *J*=2.3 Hz), 7.58 (d, 2H, *J*=2.3 Hz). ESI⁺/MS m/z 423 [M+Na]⁺. ESI⁺/MS/MS m/z 323 (100).

### Intermediate b) (2S)-2-Amino-3-(4-cyanophenyl)-N-(2-oxoazepan-3-yl)propanamide.

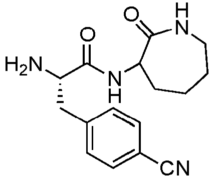

A solution of the N-BOC-protected intermediate (0.36 mmol) in 1,4-dioxane (7 mL) and 3N HCl (3.5 mL) is stirred overnight at room temperature. Then, the organic solvent is removed under reduced pressure and the aqueous solution is alkalized (pH= 14) with 5% aqueous NaOH and extracted with AcOEt (3 × 20 mL). The combined organic phases are dried over anhydrous Na₂SO₄, filtered and concentrated under reduced pressure to provide the desired compound as transparent oil that is used in the next step without further purifications (69% yield). ¹H NMR (CDCl₃): δ 1.21-1.53 (m, 2H), 1.81 (br s, 2H, exchange with D₂O), 1.87-2.09 (m, 3H), 2.81-2.91 (m, 1H), 3.19-3.33 (m, 4H), 3.63-3.72 (m, 1H), 4.46-4.50 (m, 1H), 6.11-6.18 (m, 1H), 7.34 (d, 2H, *J*= 8.19 Hz), 7.59 (d, 2H, *J*= 8.19 Hz). ESI⁺/MS m/z 323 [M+Na]⁺. ESI⁺/MS/MS m/z 207 (100) 151 (63), 64 (39).

### (2S)-3-(4-Cyanophenyl)-2-(3-(4-fluorophenyl)ureido)-N-(2-oxoazepan-3-yl)propanamide (S)-4.

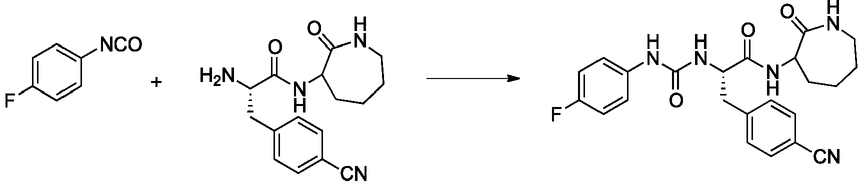

A solution of (2S)-2-amino-3-(4-cyanophenyl)-N-(2-oxoazepan-3-yl)propanamide (0.28 mmol) and 4-fluorophenylisocyanate (0.31 mmol) in anhydrous THF is stirred at room temperature overnight. Then, the solvent is removed under reduced pressure and the crude is purified by silica gel chromatography by using a mixture of CHCl₃/MeOH (95:5, v/v) as eluent. A white solid is obtained (52% yield). ¹H NMR (DMSO): δ 1.14-1.97 (m, 6H), 2.86-3.30 (m, 4H), 4.35-4.36 (m, 2H), 6.31-6.39 (m, 1H), 7.01 (t, 2H, *J*= 8.32 Hz), 7.28-7.29 (m, 2H), 7.43 (d, 2H, *J*= 7.83 Hz), 7.70-7.73 (m, 2H), 7.80-7.82 (m, 1H), 8.14-8.19 (m, 1H), 8.66 (d, 1H, *J*= 7.83 Hz). ESI⁻/MS m/z 436 [M-H]⁻. ESI⁻/MS/MS m/z 282 (14), 116 (100).

### Synthesis of the compound (S)-7

### Intermediate a) (S)-tert-Butyl (3-(4-cyanophenyl)-1-(indolin-1-yl)-1-oxopropan-2-yl)carbamate

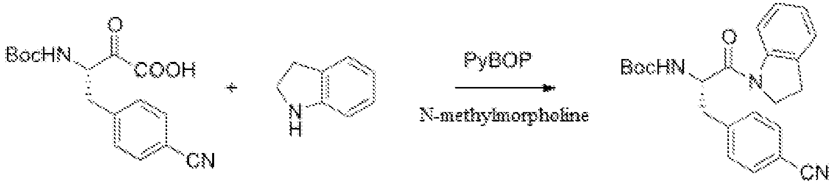

A mixture of (S)-Boc-4-CN-phenylalanine (0.25 g, 0.861 mmol), indoline (1.032 mmol), PyBOP (0.69 g, 1.29 mmol), and N-methylmorpholine (0.70 g, 6.88 mmol) in anhydrous DMF is stirred at room temperature overnight. After completion, the mixture is diluted with H₂O and extracted with AcOEt (3 × 20 mL). The combined organic phases are washed with a saturated NaCl aqueous solution, anhydrified over Na₂SO₄, filtered and concentrated under reduced pressure. The residue is chromatographed by using a mixture of CHCl₃/AcOEt (7:3, v/v) as eluent to provide the desired product as pink solid. (42% yield). ¹H NMR (CDCl3): δ 1.39 (s, 9H), 2.96-3.05 (m, 1H), 3.11-3.21 (m, 1H), 3.64 (q, 1H, *J*= 9.3 Hz), 4.19 (q, 1H, *J*= 9.3 Hz), 4.78 (t, 1H, *J*= 7.2 Hz), 5.38 (d, 1H, *J*= 8.3 Hz), 7.04-7.22 (m, 3H), 7.34 (d, 2H, *J*= 7.8 Hz), 7.55 (d, 2H, *J*= 7.8 Hz), 8.17 (d, 1H, *J*= 7.8 Hz). ESI⁺/MS m/z 414 [M+Na]⁺. ESI⁺/MS/MS m/z 314 (100).

### Intermediate b) (S)-4-(2-Amino-3-(indolin-1-yl)-3-oxopropyl)benzonitrile

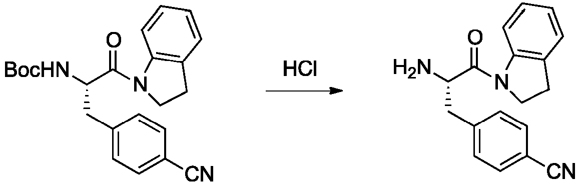

The product was prepared as described for the intermediate b of the compound (S)-4 from 0.15 g (S)-tert-Butyl (3-(4-cyanophenyl)-1-(indolin-1-yl)-1-oxopropan-2-yl)carbamate. 0.06 g white solid is obtained (38% yield). ESI⁺/MS m/z 414 [M+Na]⁺. ESI⁺/MS/MS m/z 314 (100).

### (S)-1-(3-(4-Cyanophenyl)-1-(indolin-1-yl)-1-oxopropan-2-yl)-3-(4-fluorophenyl)urea (S)-7

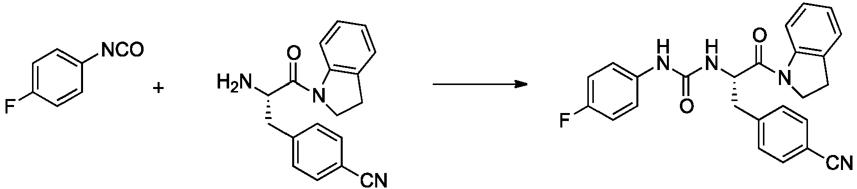

The product was prepared from (S)-4-(2-amino-3-(indolin-1-yl)-3-oxopropyl)benzonitrile (0.28 mmol) and 4-fluorenylisocyanate (0.31 mmol) as described for the compound (S)-4. The reaction crude was purified by silica gel chromatography by using a mixture of CHCl₃/AcOEt (8:2, v/v) as eluent. A white solid is obtained (49% yield). ¹H NMR (CDCl₃): δ 1.65-1.82 (m, 4H), 2.90 (dd, 1H, *J*= 7 Hz, *J*= 12.88 Hz), 3.02 (dd, 1H, *J*= 6.44 Hz, *J*= 13.47 Hz), 3.15-3.27 (m, 2H), 3.48-3.54 (m, 2H), 4.66 (q, 1H, *J*= 7.61 Hz), 6.51 (d, 1H, *J*= 7.61 Hz), 6.99-7.33 (m, 4H), 7.39 (d, 2H, *J*= 8.20 Hz), 7.73 (d, 2H, *J*= 8.20 Hz), 8.66 (s, 1H).

### Synthesis of the compound (S)-8

### Intermediate a) (S)-tert-Butyl (3-(4-cyanophenyl)-1-(6-fluoroindolin-1-yl)-1-oxopropan-2-yl)carbamate.

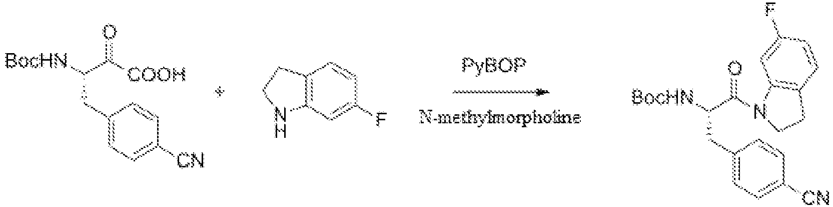

The product was synthesized from (S)-Boc-4-CN-phenylalanine (0.25 g, 0.861 mmol) and 6-fluoroindoline (1.032 mmol) as described for the intermediate a) of the compound (S)-7. The reaction crude was purified by silica gel chromatography by using an n-hexane/AcOEt mixture (1:1, v/v) as eluent. A yellow solid is obtained (37% yield) ¹H NMR (CDCl₃): δ 1.39 (s, 9H), 2.89-3.20 (m, 4H), 3.58-3.68 (m, 1H), 4.21-4.28 (m, 1H), 4.72-4.77 (m, 1H), 5.36 (d, 1H, *J*= 8.78 Hz), 6.71-6.77 (m, 1H), 7.05-7.10 (m, 1H), 7.53 (d, 2H, *J*= 8.20 Hz), 7.56 (d, 2H, *J*= 8.20 Hz), 7.91-7.95 (m, 1H). ESI⁺/MS m/z 432 [M+Na]⁺. ESI⁺/MS/MS m/z 320 (100), 135 (89), 89 (68).

### Intermediate b) (S)-4-(2-Amino-3-(6-fluoroindolin-1-yl)-3-oxopropyl)benzonitrile

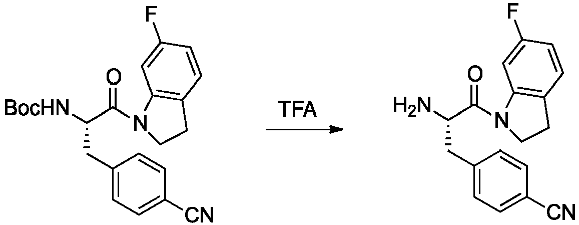

To a solution of (S)-tert-butyl (3-(4-cyanophenyl)-1-(6-fluoroindolin-1-yl)-1-oxopropan-2-yl)carbamate (0.628 mmol) in CH₂Cl₂ (5 mL) trifluoroacetic acid (1.60 mL) is added and the mixture is stirred for 5 hours at room temperature. Next, the mixture is alkalized (pH= 14) with 5% aqueous NaOH. The organic phase is separated and the aqueous phase is extracted with CH₂Cl₂ (2 × 20 mL). The combined organic phases are dried over anhydrous Na₂SO₄, filtered and concentrated under reduced pressure to provide the desired compound as white solid that is used for the next step without further purifications (97% yield). ¹H NMR (CDCl₃): δ 1.69 (br s, 2H, exchanged with D₂O), 2.86-3.16 (m, 4H), 3.68-3.84 (m, 2H), 4.12-4.21 (m, 1H), 6.73 (td, 1H, *J*= 2.34 Hz, *J*= 8.70 Hz), 7.05-7.10 (m, 1H), 7.34 (d, 2H, *J*= 8.20 Hz), 7.57 (d, 2H, *J*= 8.20 Hz), 8.00 (dd, 1H, *J*= 2.34 Hz, *J*= 10.54 Hz). ESI⁺/MS m/z 310 [M+H]⁺. ESI⁺/MS/MS m/z 145 (100), 138 (48), 128 (65).

### (S)-1-(3-(4-Cyanophenyl)-1-(6-fluoroindolin-1-yl)-1-oxopropan-2-yl)-3-(4-fluorophenyl)urea (S)-8

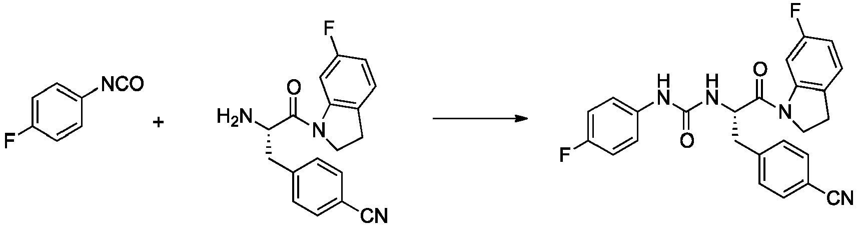

The product was prepared from (S)-4-(2-amino-3-(6-fluoroindolin-1-yl)-3-oxopropyl)benzonitrile (0.28 mmol) and 4-fluorenylisocyanate (0.31 mmol) as described for the compound (S)-4. The reaction crude was chromatographed on silica gel by using a mixture of CH₂Cl₂/EtOAc (8:2, v/v) as eluent. A white solid is obtained (57% yield). ¹H NMR (CDCl₃): δ 3.08-3.20 (m, 3H), 3.27-3.34 (m, 1H), 4.13-4.22 (m, 1H), 5.00- 5.05 (m, 8m, 1H), 1H), 6.31 (d, 1H, *J*= 8.20 Hz), 6.74-6.81 (m, 1H), 6.95-7.01 (m, 2H), 7.19-7.23 (m, 1H), 7.42-7.48 (m, 2H), 7.54 (d, 2H, *J*= 8.20 Hz), 7.68 (d, 2H, *J*= 8.20 Hz), 7.89 (dd, 1H, *J*= 2.34 Hz, *J*= 10.54 Hz), 8.20 (s, 1H). ESI⁺/MS m/z 447 [M+H]⁺. ESI⁺/MS/MS m/z 145 (100).

### Synthesis of the compound (S)-9

### Intermediate a) (S)-tert-Butyl (3-(4-cyanophenyl)-1-(5-fluoroindolin-1-yl)-1-oxopropan-2-yl)carbamate

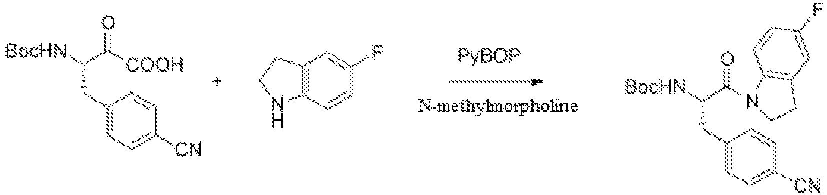

The product was synthesized from (S)-Boc-4-CN-phenylalanine (0.25 g, 0.861 mmol) and 5-fluoroindoline (1.032 mmol) as described for the intermediate a) of the compound (S)-7. The reaction crude was chromatographed on silica gel by using a mixture of CH₂Cl₂/AcOEt (8:2, v/v) as eluent. A white solid is obtained (96% yield). ¹H NMR (CDCl₃): δ 1.39 (s, 9H), 2.98-3.05 (m, 2H), 3.10-3.20 (m, 2H), 3.64 (dd, 1H, *J*= 10.27 Hz, *J*= 16.63 Hz), 4.21 (dd, 1H, *J*= 0.80 Hz, *J*= 16.64 Hz) 4.75-4.79 (m, 1H), 5.34 (br s, 1H), 6.87-6.91 (m, 2H), 7.33 (d, 2H, *J*= 7.83 Hz), 7.55 (d, 2H, *J*= 7.83 Hz), 8.13 (dd, 1H, *J*= 4.89 Hz, *J*= 7.83 Hz). ESI⁺/MS m/z 432 [M⁺Na]⁺. ESI⁺/MS/MS m/z 332 (100), 320 (80), 89 (60).

### Intermediate b) (S)-4-(2-Amino-3-(6-fluoroindolin-1-yl)-3-oxopropyl)benzonitrile

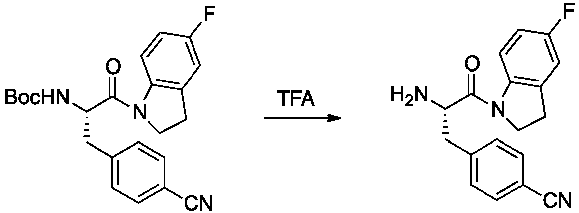

The product was prepared from (S)-tert-butyl (3-(4-cyanophenyl)-1-(5-fluoroindolin-1-yl)-1-oxopropan-2-yl)carbamate as described for the intermediate b) of the compound (S)-8. The reaction crude was chromatographed on silica gel by using an n-hexane/AcOEt mixture as eluent, with a gradient of 7:3 to 1:1 (v/v). A white solid is obtained (63% yield). ¹H NMR (CDCl₃): δ 3.02-3.10 (m, 2H), 3.12-3.80 (m, 1H), 3.70-3.80 (m, 1), 4.41-4.48 (m, 1H), 5.07-5.16 (m, 1H), 6.59 (d, 2H, *J*= 8.32 Hz), 6.81-6.84 (m, 2H), 7.10-7.15 (m, 2H), 7.16-7.25 (m, 3H), 7.37 (d, 2H, *J*= 7.83 Hz), 7.54 (d, 2H, *J*= 7.83 Hz), 8.13 (d, 1H, *J*= 7.83 Hz).

### (S)-1-(3-(4-Cyanophenyl)-1-(5-fluoroindolin-1-yl)-1-oxopropan-2-yl)-3-(4-fluorophenyl)urea (S)-9

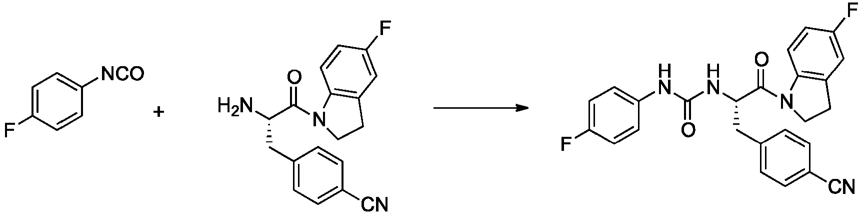

The product was prepared from (S)-4-(2-amino-3-(6-fluoroindolin-1-yl)-3-oxopropyl)benzonitrile (0.28 mmol) and 4-fluorenylisocyanate (0.31 mmol) as described for the compound (S)-4. The reaction crude was chromatographed on silica gel by using an *n*-hexane/AcOEt mixture as eluent, with a gradient of 7:3 to 1:1 (v/v). A white solid is obtained, 63% yield. ¹H NMR (CDCl₃): δ 3.02-3.10 (m, 2H), 3.12-3.80 (m, 1H), 3.70-3.80 (m, 1), 4.41-4.48 (m, 1H), 5.07-5.16 (m, 1H), 6.59 (d, 2H, *J*= 8.32 Hz), 6.81-6.84 (m, 2H), 7.10-7.15 (m, 2H), 7.16-7.25 (m, 3H), 7.37 (d, 2H, *J*= 7.83 Hz), 7.54 (d, 2H, *J*= 7.83 Hz), 8.13 (d, 1H, *J*= 7.83 Hz).

### Efficacy study of the compound (S)-16 on murine model of autism

The murine models used in the studies of the autism spectrum disorder are validated essentially on the basis of two behavioral characteristics: difficulties in the social behavior and communication and repetitive-compulsive behavior. The BTBR T + tf/J (BTBR) murine strain, which exhibits difficulty with the social approach, repetitive and compulsive behaviors and an inflammatory profile of the central nervous system, was used in the present study. In contrast, common laboratory mice of the C57BL/6 (C57) wild type (wt) strain were used as controls.

The (S)-16 molecule was administered intraperitoneally and the tests were performed in the animals both in the acute setting, *i.e.* one hour after the single administration, and after 8 consecutive days of administration. Several dosages were tested, ranging from 1 to 50 mg/kg; the pharmacologically active dose at which no adverse effects were seen following the administration for 8 consecutive days was 10 mg/kg.

The behavioral phenotype of the animals treated with (S)-16 was studied and analyzed through the use of several behavioral tests widely described in the literature and validated by the scientific community, in order to assess both the possible improvements in the repetitive compulsive behavior (*e.g. marble burying* and *self-grooming*), and the social interaction (*e.g. three-chambered social test* and *reciprocal social interactions test*).

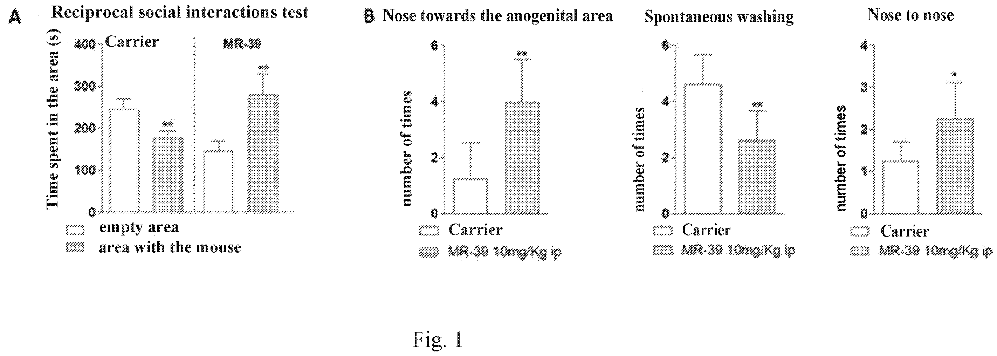

The results achieved, shown in Figure 1, demonstrate that the systemic treatment with the FPR2 (S)-16 receptor agonist at the dose of 10 mg/kg for 8 days is able to improve the social behavior in the BTBR animals in the *three-chambered social test* (Figure 1A) and in the *reciprocal social interactions test* (Figure 1B), while no improvement could be highlighted in the repetitive compulsive behavior.

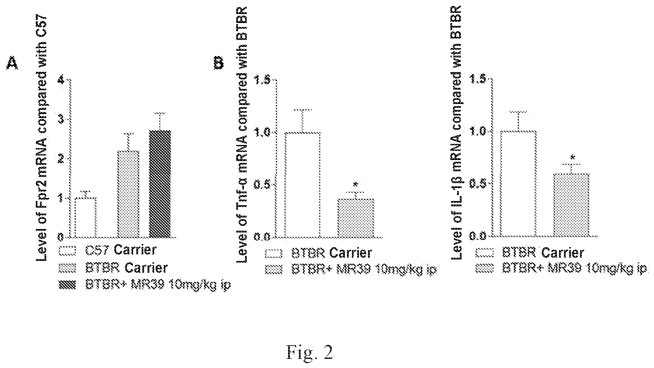

The day after the last administration, the animals were sacrificed and the brains were withdrawn for the biochemical analyses. The results of these analyses, shown in Figure 2, revealed an increase in the FPR2 levels at the central level (first graph on the left in Figure 2) and a reduction in the pro-inflammatory cytokines interleukin 1 beta (IL-1β) and tumor necrosis factor alpha TNF-α (middle and right graphs in Figure 2, respectively).

### Efficacy study of the compound (S)-16 on neuronal cultures

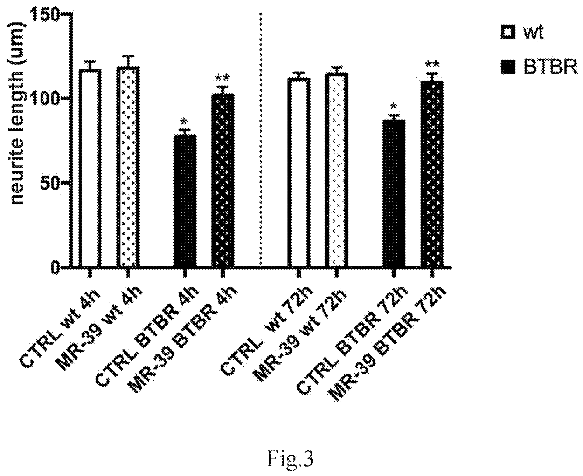

To test whether the activation of FPR2 might have an effect on the plastic responses of the neurons, cultures of postnatal hippocampal neurons were prepared. Said cultures were treated, 5 days after their preparation, with an amount equal to 10 µM of (S)-16 or with the carrier only (CTRL) for 4 and 72 hours. The cells were fixed and stained with anti-TuJ1 (neuronal selective marker) antibodies and the neurite length (µm), a parameter that is used to analyze the ability of the neurons to respond to external stimuli and pharmacological manipulations, due to their ability to modulate the length of the neuronal extensions, was measured. As shown in Figure 3, the neurite length (µm) of the hippocampal neurons of the BTBR mice under basal conditions is significantly shorter compared with that of the wt control mice (One Way ANOVA test: *p<0.001 compared with wt CTRL). Furthermore, the treatment with the (S)-16 agonist significantly stimulates the neurite elongation in the BTBR hippocampal neurons compared with those treated with the vehicle only (One Way ANOVA test: **p<0.01 compared with BTBR CTRL), whereas it does not affect the neurite outgrowth in the hippocampal neurons of the C57 mice. Such effect occurs both after a short-duration stimulation (4 hours) and after a long-duration stimulation (72 hours, *i.e.,* 3 days).

Such results suggest that the activation of FPR2 by the compound of the invention (S)-16 is able to stimulate the neurite outgrowth in the hippocampal neurons of the BTBR mice, but does not the affect the neurite length on the C57 mice.

Overall, the data obtained show that the stimulation of the FPR2 receptor may play a key role in modifying the neuronal connectivity in murine models of ASD.

Study of the effect of compounds (S)-4, (S)-5, (S)-7, (R)-8, (S)-8, (S)-9, (S)-12, (S)-15 and (S)-16 on the cell viability (LDH assay) in cultures of mouse microglial N9 cells.

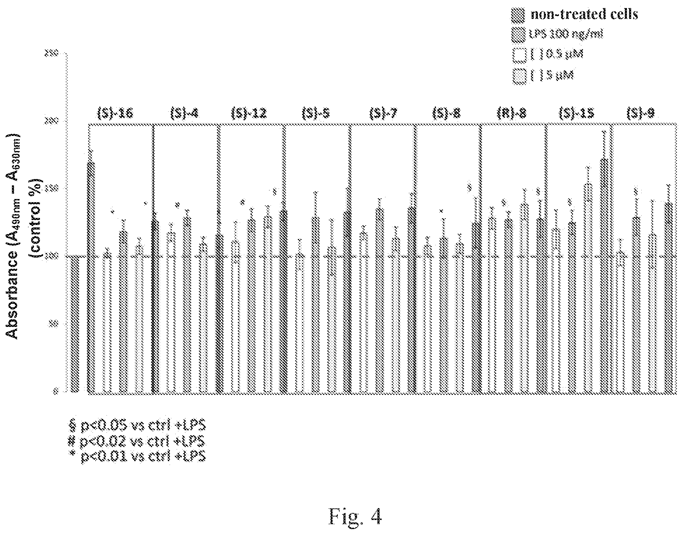

The neuroprotective activity of the compounds (S)-4, (S)-5, (S)-7, (R)-8, (S)-8, (S)-9, (S)-12, (S)-15 and (S)-16 was assessed by determining its effect on the cell viability both under basal conditions and following stimulation with bacterial lipopolysaccharide (LPS) toxin. The mouse microglial N9 cells were seeded in a 96-well plate (2×10⁴ cells/well) by using RPMI supplemented with 1% fetal bovine serum (FBS) as culture medium. The cells were incubated for 24 hours with different concentrations of the selected compounds (0.5-5 µM) to assess the potential cytotoxic effect of the compounds. The control cells were treated with the carrier only. To quantify the cell mortality, the level of lactate dehydrogenase (LDH) release after 24 hours of treatment was determined. The supernatant of the cell cultures was taken and incubated for 20 minutes at room temperature with the appropriate mixture of reagents according to the instructions of the supplier of the assessment kit (Cytotoxicity Detection Kit, Roche, Germany). The LDH release activity was determined as conversion of lactate to pyruvate by a colorimetric test (λ= 490 nm, Infinite 200 PRO Detector, TECAN, Switzerland). The LDH release activity is proportional to the number of damaged cells. The data obtained (Figure 4) show that the compounds do not increase the LDH release activity and, therefore, do not cause cellular damage. Subsequently, the cells were pre-incubated with different concentrations of the selected compounds (0.5-5 µM) and then stimulated with LPS (100 ng/mL) for 24 hours. The control cells were treated with the carrier only. The cell viability at the end of 24 hours was assessed as previously described. The data obtained (Figure 4) show that the compounds have a neuroprotective effect since they are able to reduce the cell mortality effect caused by the stimulation with LPS.

Study of the anti-inflammatory effect of the compounds (S)-4, (S)-7, (S)-8, (S)-9, and (S)-16 on cultures of mouse microglial N9 cells.

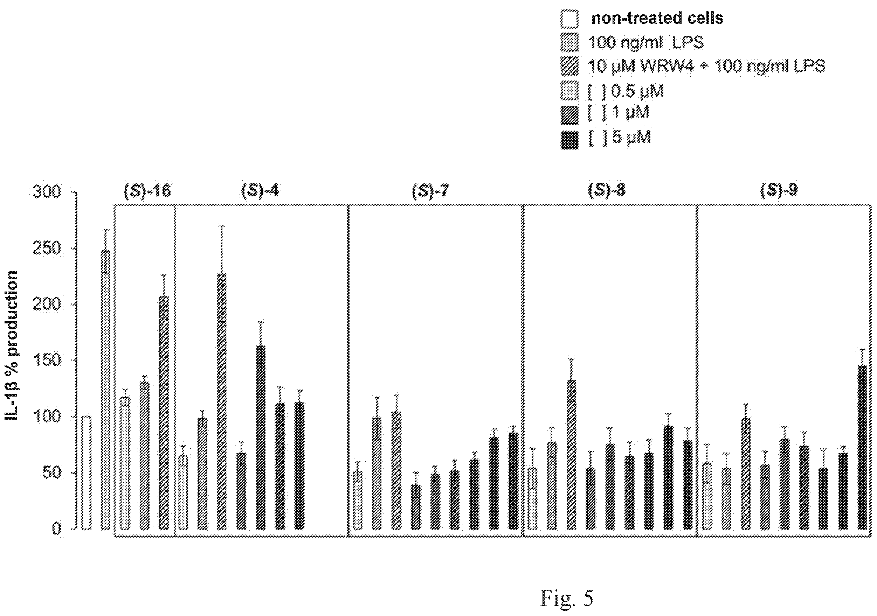

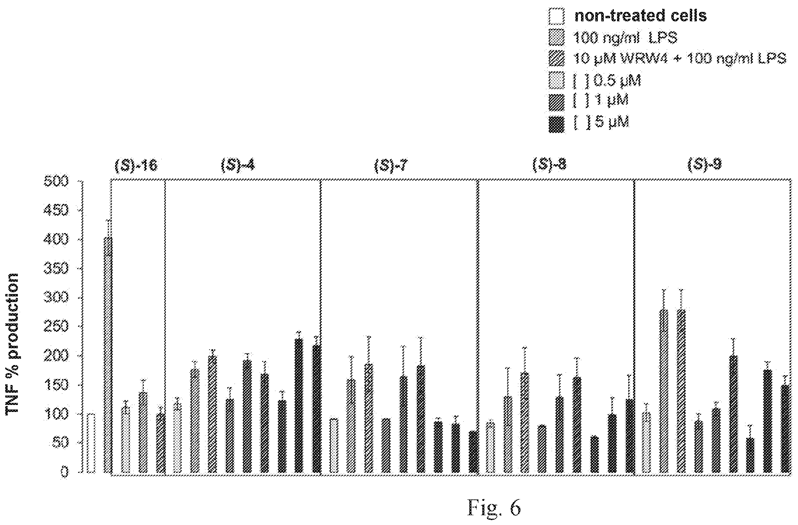

The anti-inflammatory effect of the compounds (S)-4, (S)-7, (S)-8, (S)-9 and (S)-16 was determined by assessing its ability to affect the release of the pro-inflammatory cytokines IL-1β and TNF-α both in the cells under basal conditions and after stimulation with LPS. The mouse microglial N9 cells were seeded in a 96-well plate (2×10⁴ cells/well) by using Iscove supplemented with 10% FBS as culture medium. The cells were then treated with different concentrations of the selected compounds (0.5-5 µM) for 24 hours. The supernatant was then collected and the IL-1β and TNF-α levels were measured by using specific ELISA kits following the instructions of the supplier (R&D Systems, USA). The data obtained, depicted in Figure 5 for IL-1β and in Figure 6 for TNF-α, show that the compounds analyzed do not exert a pro-inflammatory effect, as they do not cause increased release of the two cytokines. Subsequently, the cells were pre-incubated with different concentrations of the selected compounds (0.5-5 µM) and then stimulated with LPS (100 ng/mL) for 24 hours. The control cells were treated with the carrier only. The IL-1β and TNF-α levels were determined as previously described. The data obtained, depicted in Figure 5 for IL-1β and in Figure 6 for TNF-α, show that the compounds exert an anti-inflammatory effect as they are able to reduce the release of inflammatory cytokines, which is caused by the stimulation with LPS. The specificity of the observed effect was assessed by co-incubating the cells with the FPR2 antagonist WRW4.

## Claims

1. A compound of formula (I) 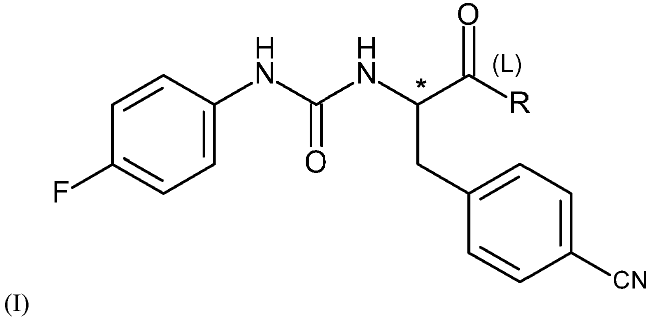 wherein R is selected from one of the following groups, wherein (a) denotes the nitrogen atom engaged in bonding to the compound of formula (I) and (L) denotes the covalent bond binding the compound of formula (I) to each group R, 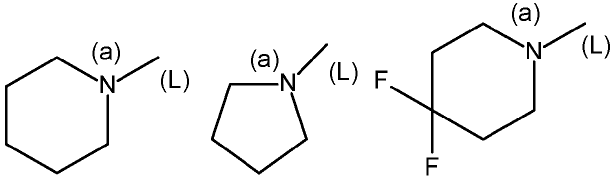 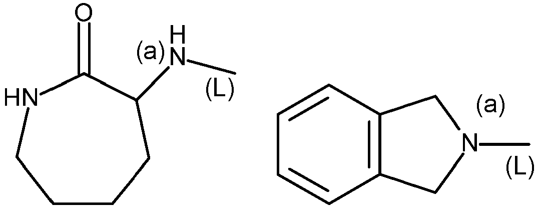 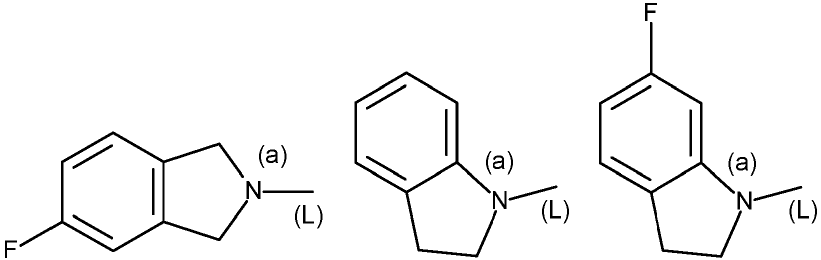 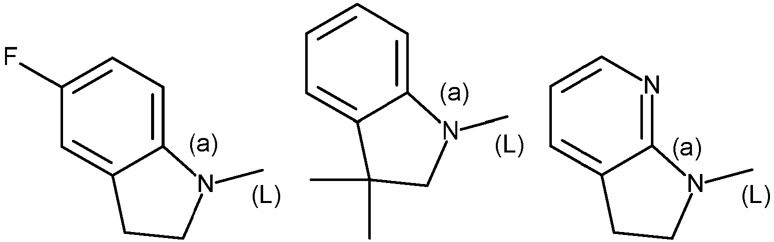 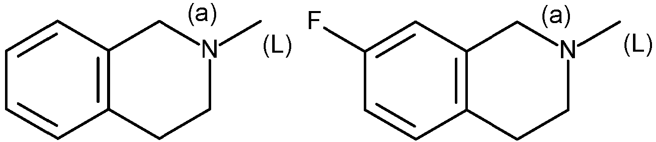 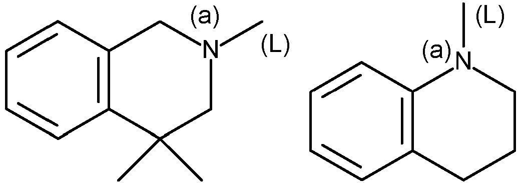 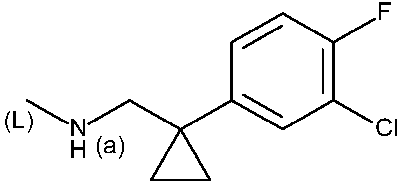 and the chiral carbon, denoted by the asterisk, may be in the S and/or R configuration.

2. Compounds (S)-4, (S)-7, (S)-8, (S)-9 and (S)-16 according to claim 1 of formula:

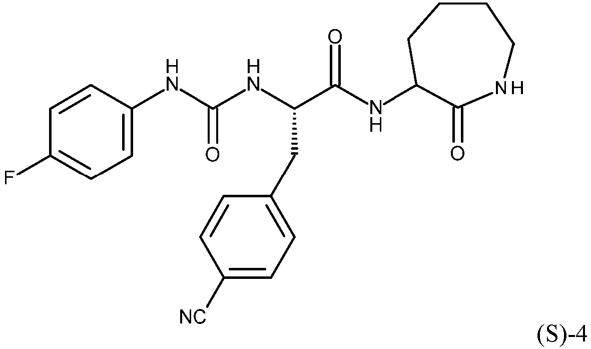

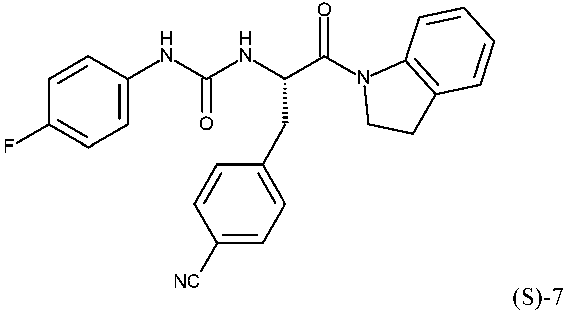

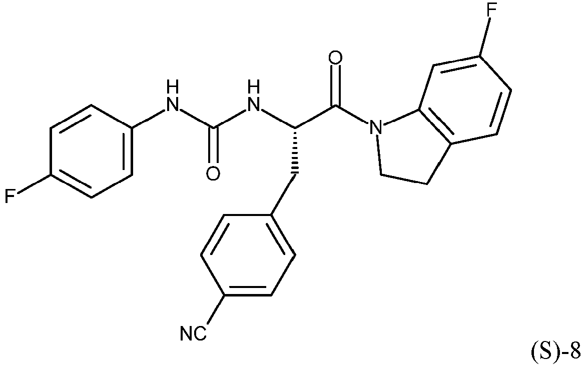

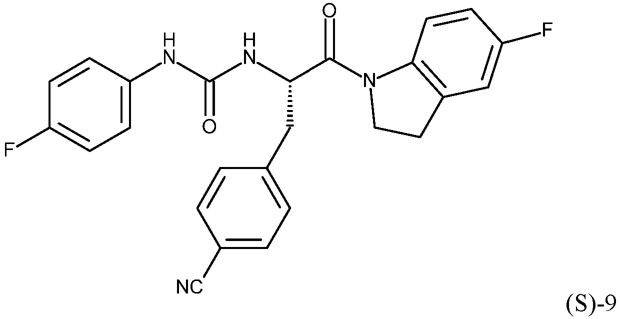

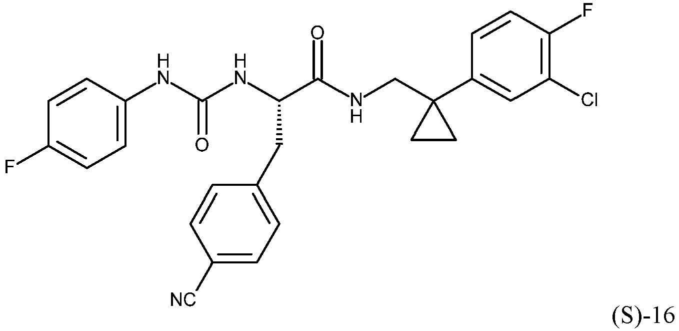

3. The compound of formula (I) according to claim 1 for its use as a medicament.

4. The compounds according to claim 2 for their use as a medicament.

5. The compound of formula (I) according to claim 1 for its use as an FPR2 receptor agonist.

6. The compounds according to claim 2 for their use as an FPR2 receptor agonist.

7. The compound of formula (I) according to claim 1 for its use in the treatment of the autism spectrum disorder.

8. The compounds according to claim 2 for their use in the treatment of the autism spectrum disorder.

9. A pharmaceutical composition comprising a compound of formula (I) according to claim 1 or a compound according to claim 2, at least one pharmaceutically acceptable vehicle and possibly at least another active compound.

10. The pharmaceutical composition according to claim 9 for its use in the treatment of conditions involving poor activation of the FPR2 receptor, in particular for its use in the treatment of the autism spectrum disorder.

## Patentansprüche

1. Eine Verbindung der Formel (I), 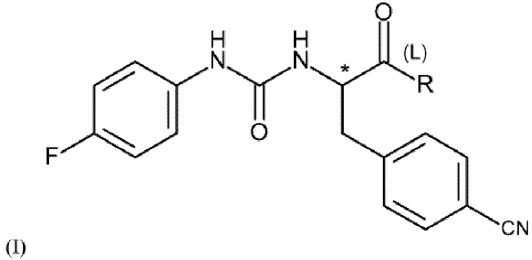 wobei R aus einer der folgenden Gruppen ausgewählt ist, wobei (a) das Stickstoffatom bezeichnet, das an der Bindung mit der Verbindung der Formel (I) beteiligt ist, und (L) die kovalente Bindung bezeichnet, die die Verbindung der Formel (I) zu jeder Gruppe R bindet, 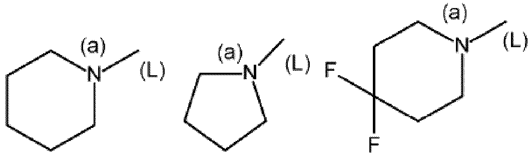 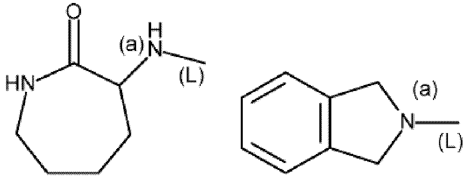 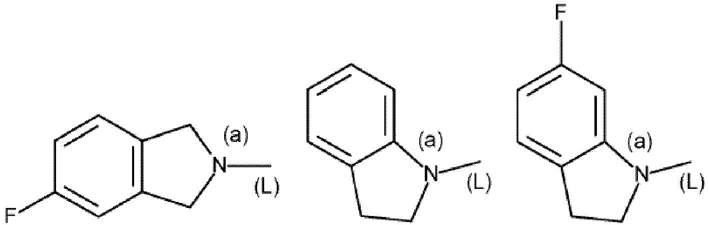 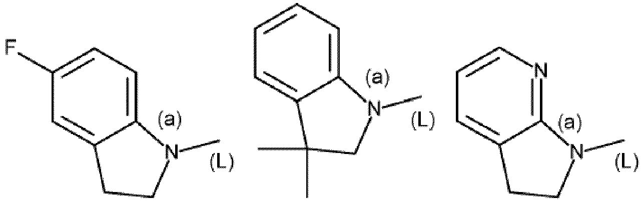 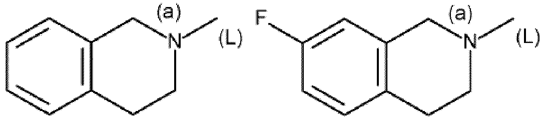 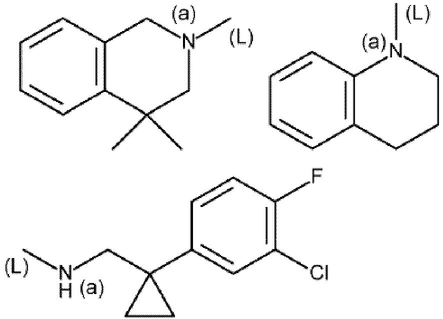 und das mit einem Stern gekennzeichnete chirale Kohlenstoffatom in der S- und/oder R-Konfiguration vorliegen kann.

2. Verbindungen (S)-4, (S)-7, (S)-8, (S)-9 und (S)-16 gemäß Anspruch 1 der Formel:

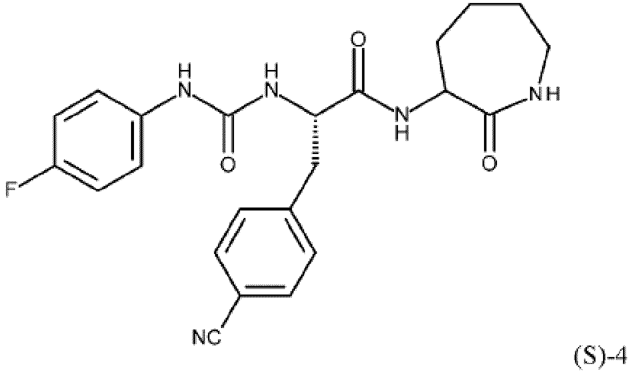

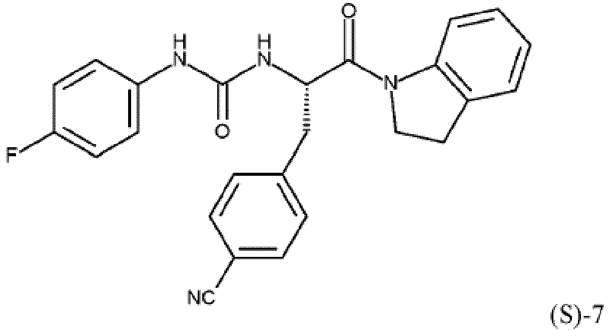

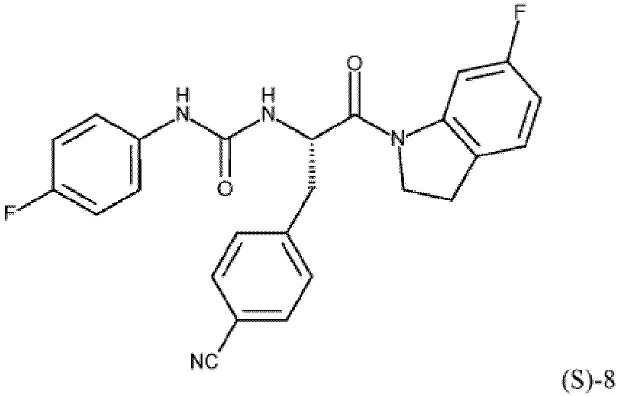

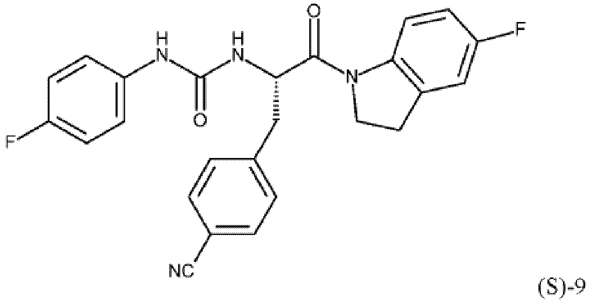

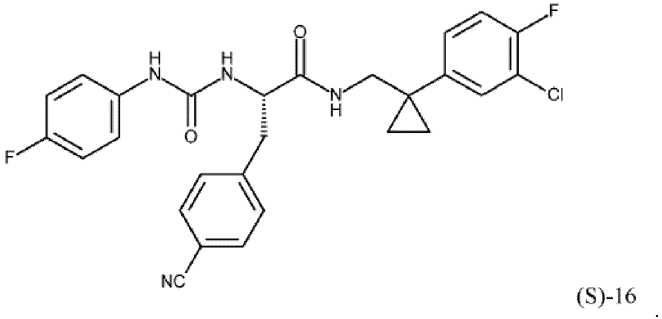

3. Die Verbindung der Formel (I) gemäß Anspruch 1 zur Verwendung als Arzneimittel.

4. Die Verbindungen gemäß Anspruch 2 zur Verwendung als Arzneimittel.

5. Die Verbindung der Formel (I) gemäß Anspruch 1 zur Verwendung als FPR2-Rezeptoragonist.

6. Die Verbindungen gemäß Anspruch 2 zur Verwendung als FPR2-Rezeptoragonist.

7. Die Verbindung der Formel (I) gemäß Anspruch 1 zur Verwendung bei der Behandlung von Autismus-Spektrum-Störungen.

8. Die Verbindungen gemäß Anspruch 2 zur Verwendung bei der Behandlung von Autismus-Spektrum-Störungen.

9. Eine pharmazeutische Zusammensetzung, enthaltend eine Verbindung der Formel (I) gemäß Anspruch 1 oder eine Verbindung gemäß Anspruch 2, mindestens ein pharmazeutisch annehmbares Vehikel und möglicherweise mindestens eine weitere aktive Verbindung.

10. Die pharmazeutische Zusammensetzung nach Anspruch 9 zur Verwendung in der Behandlung von Störungen, bei denen der FPR2-Rezeptor unzureichend aktiviert ist, insbesondere für die Behandlung von Autismus-Spektrum-Störungen.

## Revendications

1. Composé de la formule (I) 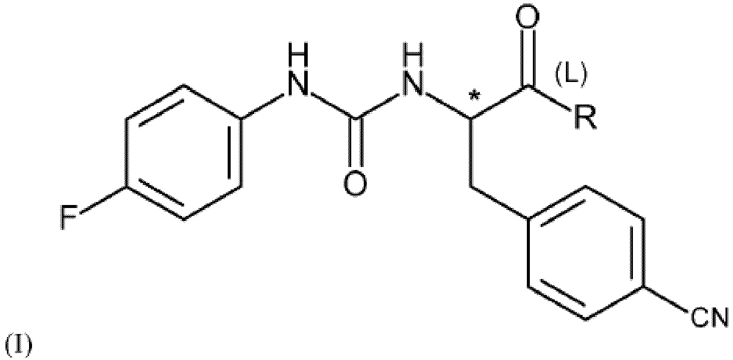 dans lequel R est choisi parmi l'un des groupes suivants, dans lequel (a) désigne l'atome d'azote engagé dans la liaison avec le composé de formule (I) et (L) désigne la liaison covalente liant le composé de formule (I) à chaque groupe R, 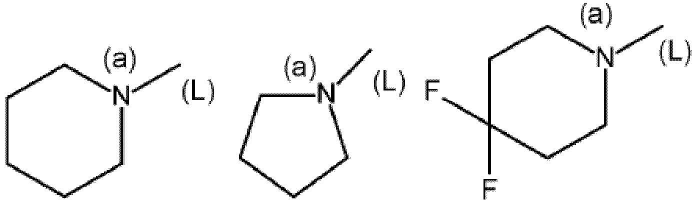 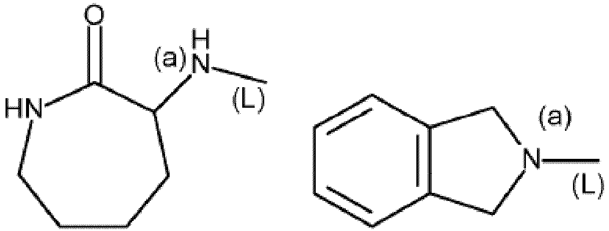 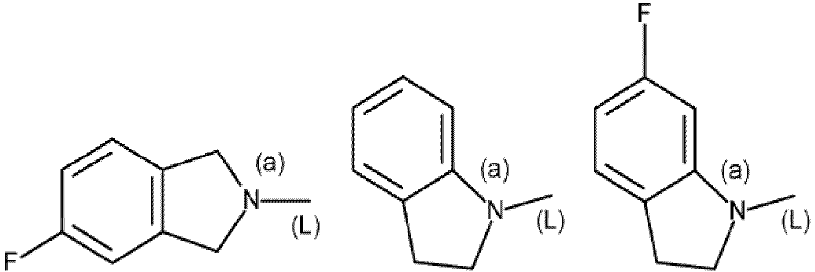 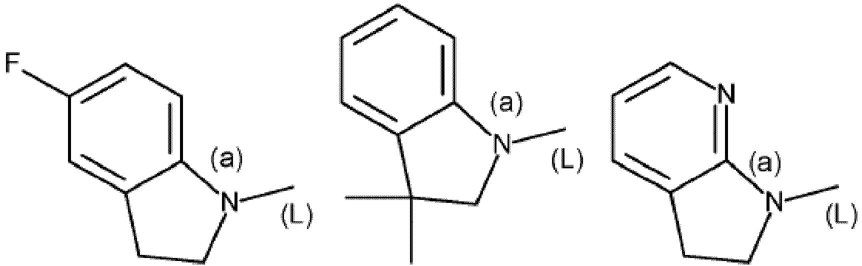 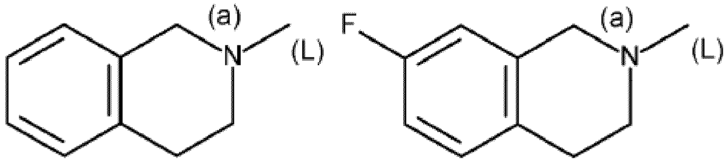 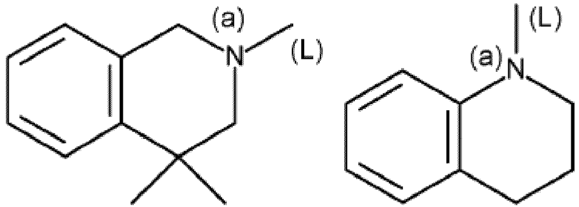 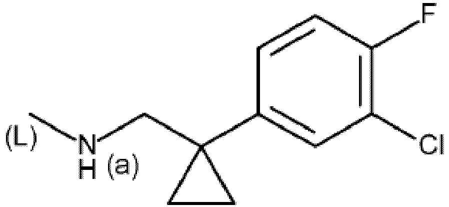 et le carbone chiral, indiqué par un astérisque, peut se trouver dans la configuration S et/ou R

2. Composés (S)-4, (S)-7, (S)-8, (S)-9 et (S)-16 selon la revendication 1 de formule :

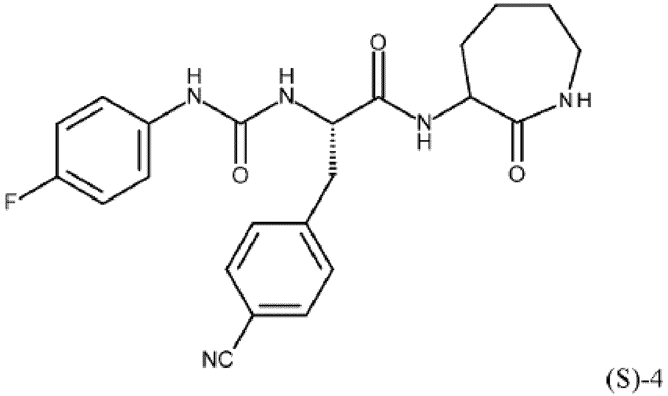

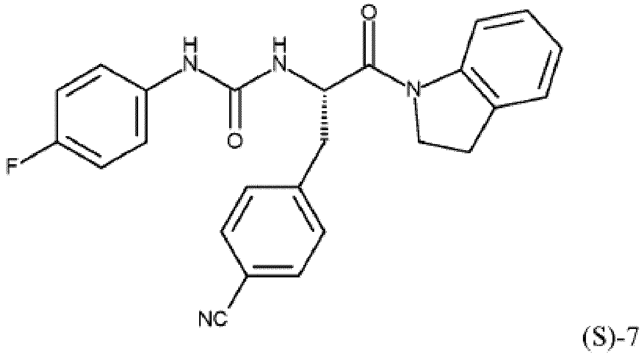

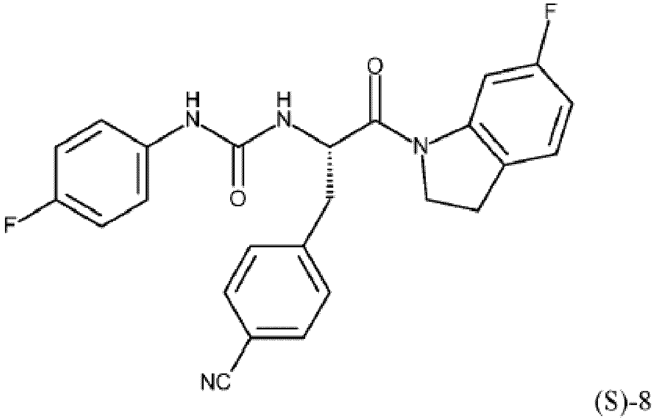

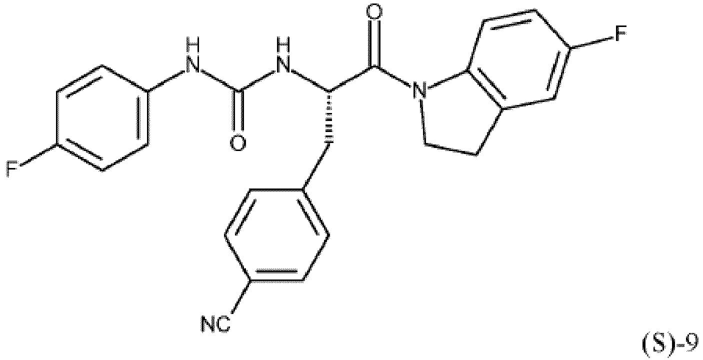

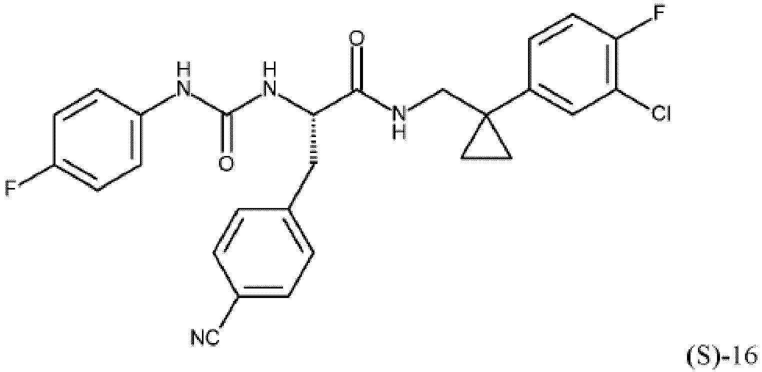

3. Composé de formule (I) selon la revendication 1 pour son utilisation en tant que médicament.

4. Composés selon la revendication 2 pour leur utilisation en tant que médicament.

5. Composé de formule (I) selon la revendication 1 pour son utilisation en tant qu'agoniste du récepteur FPR2.

6. Composés selon la revendication 2 pour leur utilisation en tant qu'agoniste du récepteur FPR2.

7. Composé de formule (I) selon la revendication 1 pour son utilisation dans le traitement des troubles du spectre autistique.

8. Composés selon la revendication 2 pour leur utilisation dans le traitement des troubles du spectre autistique.

9. Composition pharmaceutique comprenant un composé de formule (I) selon la revendication 1 ou un composé selon la revendication 2, au moins un véhicule pharmaceutiquement acceptable et éventuellement au moins un autre composé actif.

10. Composition pharmaceutique selon la revendication 9 pour son utilisation dans le traitement des conditions impliquant une faible activation du récepteur FPR2, en particulier pour son utilisation dans le traitement des troubles du spectre autistique.
